# EUROPÄISCHE PATENTSCHRIFT

(11) **EP 3 274 045 B1**
(45) Veröffentlichungstag und Bekanntmachung des Hinweises auf die Patenterteilung: **17.06.2020**
(21) Anmeldenummer: 16709379.8
(22) Anmeldetag: 09.03.2016
(51) Int. Cl.: A61M 39/22, F16K 11/00, A61M 39/10, F16K 11/085, F16K 15/14

(54) **MEDIZINISCHE FLUIDSTEUERVORRICHTUNG FÜR EIN MEDIZINISCHES FLUIDLEITUNGSSYSTEM**
MEDICAL FLUID CONTROL DEVICE FOR A MEDICAL FLUID LINE SYSTEM
DISPOSITIF MÉDICAL DE COMMANDE DE FLUIDE POUR UN SYSTÈME MÉDICAL DE CONDUITES DE FLUIDE

(30) Priorität: 26.03.2015 DE 102015205517
(43) Veröffentlichungstag der Anmeldung: 31.01.2018
(73) Patentinhaber: B. Braun Melsungen AG, 34212 Melsungen (DE)
(72) Erfinder: KUNSCHAK, Ralf, 6130 Willisau (CH); BIERI, Sebastian, 6170 Schüpfheim (CH); SCHNEIDER, Martin, 3604 Thun (CH); DUDHANE, Mayur, 6182 Escholzmatt (CH); EISEN, Frank, 6182 Escholzmatt (CH); KOBEL, Fritz, 3325 Hettiswil (CH)
(74) Vertreter: Patentanwälte Ruff, Wilhelm, Beier, Dauster & Partner mbB
(86) Internationale Anmeldenummer: PCT/EP2016/055024
(87) Internationale Veröffentlichungsnummer: WO 2016/150707

(56) Entgegenhaltungen:
- EP-A1- 2 808 586
- EP-A2- 0 410 898
- WO-A1-2007/084214
- WO-A1-2013/146753
- WO-A1-2014/049810

## Beschreibung

Die Erfindung betrifft eine medizinische Fluidsteuervorrichtung für ein medizinisches Fluidleitungssystem mit einem Fluidströmungsgehäuse, das mit wenigstens einem Verbindungsport zur Verbindung mit einer weiteren Funktionskomponente des Fluidleitungssystems versehen ist, und das einen Zuspritzport aufweist, der mit einem öffnungsfähigen Verschlusselement versehen ist, sowie mit einem Stellorgan, das in dem Fluidströmungsgehäuse beweglich gelagert ist und Strömungskanalabschnitte aufweist, die abhängig von einer Stellposition des Stellorgans dem wenigstens einen Verbindungsport und/oder dem Zuspritzport für eine Fluiddurchströmung zustellbar sind.

Eine derartige medizinische Fluidsteuervorrichtung ist aus der US 7,984,730 B2 bekannt. Die bekannte medizinische Fluidsteuervorrichtung ist als Dreiwegehahn für ein medizinisches Infusionssystem ausgeführt. Der Dreiwegehahn weist zwei Verbindungsporte sowie einen Zuspritzport auf, die alle einstückig an einem Fluidströmungsgehäuse angeformt sind. Der Zuspritzport ist mit einem öffnungsfähigen Verschlusselement in Form einer mit einem Luer-Lock-Anschluss montierbaren Verschlusskappe oder in Form einer elastisch flexiblen Verschlussmembran versehen. Die Verschlusskappe kann von dem Zuspritzport abgedreht und entfernt werden. Die Verschlussmembran ist öffnungsfähig, indem sie im Bereich eines stirnseitigen Durchtrittsschlitzes elastisch geöffnet werden kann. Eine Öffnung der Verschlussmembran erfolgt insbesondere durch das Ansetzen einer Spritze. Das Fluidströmungsgehäuse weist ein im Fluidströmungsgehäuse drehbeweglich gelagertes Stellorgan auf, das mit Strömungskanalabschnitten versehen ist, die je nach Stellung des Stellorgans mit den beiden Verbindungsporten oder mit wenigstens einem Verbindungsport und dem Zuspritzport in Fluidverbindung, d.h. in Durchströmungsverbindung, stehen. Bei verschlossenem Verschlusselement, d.h. bei verschlossenem Zuspritzport, ist es so möglich, eine Fluiddurchströmung im Bereich der Verbindungsporte über den Zuspritzport umzuleiten und so eine Durchspülung einer Innenseite des Zuspritzports zu bewirken. Die ungewünschte Ablagerung von Medikamentenresten im Zuspritzport kann hierdurch vermieden werden.

Weitere medizinische Fluidsteuerungsvorrichtungen werden in WO 2014/049810 A1 sowie WO 2013/146753 A1 offenbart.

Aufgabe der Erfindung ist es, eine medizinische Fluidsteuervorrichtung der eingangs genannten Art zu schaffen, die eine besonders genaue Medikamentendosierung für einen Patienten ermöglicht.

Diese Aufgabe wird dadurch gelöst, dass wenigstens einem Strömungskanalabschnitt des Stellorgans und/oder dem Zuspritzport wenigstens ein mechanisches Strömungsbeschleunigungsmittel zugeordnet ist, das bei einer Fluiddurchströmung eine innenseitige Spülung des Zuspritzports unterstützt. Dadurch wird gegenüber dem Stand der Technik eine weiter verbesserte Spülung der Innenseite des Zuspritzports erzielt. Bei einer Medikamentenzugabe im Bereich des Zuspritzports ist erfindungsgemäß somit gewährleistet, dass keine Medikamentenreste im Bereich des Zuspritzports verbleiben, so dass eine über den Zuspritzport zugeführte Medikamentendosierung in vollem Umfang in eine Patientenleitung des medizinischen Fluidleitungssystems gelangen kann und demzufolge eine hochgenaue Dosierung des Medikaments beim Patienten ermöglicht. Zudem werden durch die permanente Spülung des lateralen Verbindungsports/Zuspritzports Ablagerungen vermieden. Die mechanischen Strömungsbeschleunigungsmittel bewirken eine Geschwindigkeitserhöhung der durch den wenigstens einen Verbindungsport und den Zuspritzport geleiteten Fluidströmung, wodurch die Fluidströmung zwangsläufig eine höhere Spülleistung im Bereich der Innenseite des Zuspritzports bewirken kann. Als mechanische Strömungsbeschleunigungsmittel sind mechanische Strömungsleitmittel vorgesehen, die eine Verengung eines Strömungsquerschnitts für die Fluiddurchströmung in dem wenigstens einen Strömungskanalabschnitt erzielen. Das Strömungsbeschleunigungsmittel kann eine Düsenfunktion aufweisen. Alternativ oder ergänzend ist es vorgesehen, dass wenigstens ein Strömungsbeschleunigungsmittel durch Drallerzeugungsmittel an Wandbereichen des wenigstens einen Strömungskanalabschnitts zu bilden, die auf die Fluiddurchströmung eine Drallwirkung ausüben und hierdurch ebenfalls eine Beschleunigung insbesondere von wandungsseitigen Fluidströmungsbereichen erzielen. Die erfindungsgemäße Lösung eignet sich in besonders vorteilhafter Weise für den Einsatz bei einem medizinischen Fluidleitungssystem in Form eines Infusionssystems. Besonders vorteilhaft ist die erfindungsgemäße Lösung einsetzbar bei einem Infusionssystem zur Verabreichung von Zytostatika. Das öffnungsfähige Verschlusselement im Sinne der Erfindung ist vorzugsweise entweder als über eine Luer-Lock-Verbindung mit dem Zuspritzport verbindbare Verschlusskappe oder als elastisch flexible Verschlussmembran ausgeführt, die in den Zuspritzport integriert ist.

Gemäß der Erfindung ist das mechanische Strömungsbeschleunigungsmittel durch einen in den Strömungskanalabschnitt und/oder den Zuspritzport integrierten Rohrabschnitt gebildet, dessen Strömungsquerschnitt kleiner ist als ein Strömungsquerschnitt eines stromaufwärts benachbarten Strömungskanalabschnitts des Stellorgans oder des Zuspritzports oder des Verbindungsports. Hierdurch ergibt sich eine Rohr-in-Rohr-Ausführung, die aufgrund des reduzierten Strömungsquerschnitts des Rohrabschnitts eine Beschleunigung der Fluiddurchströmung gewährleistet. Falls der Rohrabschnitt freiliegend innerhalb des Strömungskanalabschnittes positioniert ist, wird eine Rückführung der Fluiddurchströmung außenseitig an dem Rohrabschnitt zu einem entsprechend strömungsabwärts liegenden Verbindungsport ermöglicht. Falls gemäß einer Variante dieser Ausgestaltung sowohl in dem Strömungskanalabschnitt als auch in dem Zuspritzport jeweils ein Rohrabschnitt vorgesehen ist, so sind die - je nach Stellung des Stellorgans - benachbarten Rohrabschnitte vorzugsweise koaxial zueinander ausrichtbar. Die benachbarten Rohrabschnitte weisen vorzugsweise identische Rohrquerschnitte auf. Der wenigstens eine Rohrabschnitt dient dazu, die Fluiddurchströmung mit erhöhter Strömungsgeschwindigkeit in das Innere des Zuspritzports hineinzuleiten, sobald das Stellorgan eine entsprechende Stellposition eingenommen hat.

In Ausgestaltung der Erfindung ist der Rohrabschnitt einstückig in dem Strömungskanalabschnitt des Stellorgans oder dem Zuspritzport des Fluidströmungsgehäuses ausgeformt. Dabei ist der Rohrabschnitt vorzugsweise über angeformte Radial- oder Umfangsrippen mit einer entsprechenden Innenwandung des Strömungskanalabschnitts oder des Zuspritzports verbunden.

Weiterhin gemäß der Erfindung ist zwischen einer Außenwandung des Rohrabschnitts und einer Wandung des Strömungskanalabschnitts oder einer Innenwandung des Zuspritzports ein eine Fluiddurchströmung ermöglichender Ringspalt vorgesehen. Der Ringspalt ist vorzugsweise als Teilringabschnitt nur über einen Teilbereich eines Umfangs des Rohrabschnitts vorgesehen, um die einstückige Verbindung des Rohrabschnitts mit wenigstens einer Wandung des Strömungskanalabschnitts oder einer Innenwandung des Zuspritzports nicht zu beeinträchtigen.

In weiterer Ausgestaltung der Erfindung ist eine Innenwandung des Rohrabschnitts mit Drallerzeugungsgeometrien versehen, die auf die Fluiddurchströmung einwirken. Die Drallerzeugungsgeometrien, die vorzugsweise einstückig im Bereich der Innenwandung des Rohrabschnitts angeformt sind, ermöglichen weiter verbesserte Beschleunigungen der Fluiddurchströmung. Die Drallerzeugungsgeometrien unterbrechen vorzugsweise eine laminare Strömung der Fluiddurchströmung an der Innenwandung des Rohrabschnitts und ermöglichen so eine Strömungsbeschleunigung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in perspektivischer Explosionsdarstellung eine Ausführungsform einer erfindungsgemäßen medizinischen Fluidsteuervorrichtung in Form eines Dreiwegehahns,
- Fig. 2: eine Querschnittsdarstellung des Dreiwegehahns nach Fig. 1 in einer ersten Funktionsstellung,
- Fig. 3 bis 5: Querschnittsdarstellungen des Dreiwegehahns nach den Fig. 1 und 2 in weiteren Funktionsstellungen,
- Fig. 6: in perspektivischer Darstellung ein Stellorgan des Dreiwegehahns nach den Fig. 1 bis 5,
- Fig. 7: einen Querschnitt durch das Stellorgan nach Fig. 6 auf Höhe von Strömungskanalabschnitten des Stellorgans,
- Fig. 8: einen Längsschnitt durch das Stellorgan nach den Fig. 6 und 7,
- Fig. 9: einen Querschnitt durch eine weitere Ausführungsform eines Stellorgans ähnlich den Fig. 6 und 7,
- Fig. 10: einen Längsschnitt durch das Stellorgan nach Fig. 9 gemäß dem Längsschnitt nach Fig. 8,
- Fig. 11: eine weitere Ausführungsform einer erfindungsgemäßen medizinischen Fluidsteuervorrichtung in einem Querschnitt,
- Fig. 12: eine weitere Ausführungsform einer erfindungsgemäßen medizinischen Fluidsteuervorrichtung ähnlich Fig. 2,
- Fig. 13: eine teilweise geschnittene Seitenansicht der medizinischen Fluidsteuervorrichtung nach Fig. 12 entlang der Schnittlinie XIII - XIII in Fig. 12 und
- Fig. 14: eine perspektivische Darstellung der medizinischen Fluidsteuervorrichtung gemäß den Fig. 12 und 13 unter Weglassung einer Verschlussmembran im Bereich eines Zuspritzports.

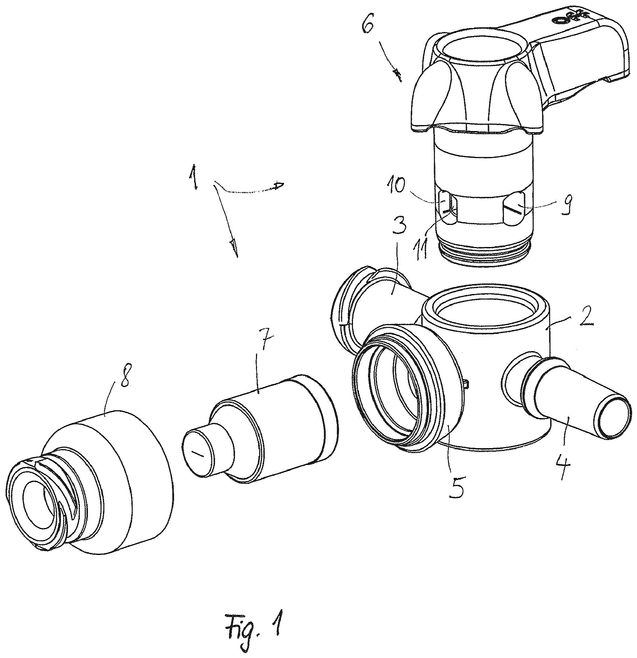

Eine medizinische Fluidsteuervorrichtung gemäß den Fig. 1 bis 8 ist als Dreiwegehahn ausgeführt und ist für den Einsatz bei einem medizinischen Fluidleitungssystem in Form eines Infusionssystems vorgesehen. Der Dreiwegehahn 1 weist ein aus Kunststoff, vorzugsweise aus Polyamid oder Polycarbonat, bestehendes Fluidströmungsgehäuse 2 auf, das hohlzylinderartig gestaltet ist. In dem Fluidströmungsgehäuse 2 ist ein Stellorgan 6 verdrehbar gelagert, das auch als Küken bezeichnet wird. Das Stellorgan 6 ist im Bereich einer Oberseite (Fig. 1) mit einem Betätigungsgriff versehen, um ein manuelles Verdrehen des Stellorgans 6 innerhalb des Fluidströmungsgehäuses 2 zwischen unterschiedlichen Funktionsstellungen zu ermöglichen. Im Bereich einer Unterseite ist das Stellorgan 6 mit nicht näher bezeichneten Rastmitteln versehen, um das Stellorgan 6 axial im Fluidströmungsgehäuse 2 zu sichern. Derartige Rastmittel zur Sicherung des Stellorgans 6 sind durch die US 6 536 742 B2 der Anmelderin bekannt.

Das Fluidströmungsgehäuse 2 weist in einer Radialebene - relativ zu einer Drehachse des Stellorgans 6 in dem Fluidströmungsgehäuse 2 gesehen - drei in unterschiedlichen Richtungen radial von dem Fluidströmungsgehäuse 2 abragende Ports auf, von denen zwei einander diametral gegenüberliegende Ports als Verbindungsports 3, 4 und ein rechtwinklig zu diesen Verbindungsports 3, 4 abragender Port als Zuspritzport 5 gestaltet sind. Sowohl die Verbindungsports 3, 4 als auch der Zuspritzport 5 sind einstückig an dem Fluidströmungsgehäuse 2 angeformt. Der Verbindungsport 3 ist an seinem relativ zum Fluidströmungsgehäuse 2 abliegenden Stirnendbereich mit außenliegenden Luer-Lock-Profilierungen für die Verbindung mit einem komplementären Luer-Lock-Anschlussteil versehen, das Teil einer weiteren Funktionskomponente des medizinischen Fluidleitungssystems ist. Der Verbindungsport 4 dient zur Verbindung mit einer Patientenleitung, d.h. einer Fluidleitung, die intravenös oder parenteral oder in anderer Art und Weise mit einem entsprechenden Patienten verbindbar ist.

Dem Zuspritzport 5 ist ein öffnungsfähiges Verschlusselement in Form einer elastisch flexiblen Verschlussmembran 7 zugeordnet, die becherartig in einem Aufnahmegehäuse 8 positioniert ist, das in montiertem Zustand fest mit dem Zuspritzport 5 verbunden ist und demzufolge einen Teilabschnitt des Zuspritzports bildet. Das Aufnahmegehäuse 8 ist an seinem zu dem Fluidströmungsgehäuse 2 abliegenden Stirnendbereich mit weiblichen Luer-Lock-Anschlussprofilierungen versehen, um den Anschluss einer komplementären Luer-Lock-Komponente zu ermöglichen, die insbesondere als Teil einer Spritze gestaltet ist.

Alternativ ist bei einer nicht dargestellten Ausführungsform der Erfindung vorgesehen, statt eines öffnungsfähigen Verschlusselements am Aufnahmegehäuse einen weiblichen Luer-Lock-Anschluss vorzusehen, der mit einem männlichen Luer-Anschluss einer Spritze durch einfaches axiales Einstecken des männlichen Luer-Anschlusses zusammenwirken kann.

Über den Zuspritzport 5 können der Patientenleitung Fluide in Form von Medikamenten zudosiert werden. Hierzu wird eine entsprechende Spritze oder eine andere Funktionskomponente mittels der Luer-Lock-Anschlüsse mit dem Zuspritzport 5 verbunden, wodurch zwangsläufig die Verschlussmembran 7 elastisch deformiert wird und in ihren Öffnungszustand überführt wird. Nach dem Zuspritzen einer entsprechenden Medikamentendosierung kann die Spritze oder eine entsprechende andere Funktionskomponente von dem Zuspritzport 5 wieder entfernt werden, wodurch die Verschlussmembran 7 wieder in ihre Schließstellung zurückbewegt wird.

Um nach dem Entfernen einer entsprechenden Funktionskomponente von dem Zuspritzport 5 Medikamentenreste, die sich noch im Bereich der Innenseite des Zuspritzports 5 befinden, herauszuspülen, wird eine Fluiddurchströmung, die von einer permanent mit dem Verbindungsport 3 verbundenen Funktionskomponente aus durch den Dreiwegehahn 1 hindurchgeleitet wird, in den Zuspritzport 5 hinein umgeleitet und anschließend über den Verbindungsport 4 zur Patientenleitung weitergeführt.

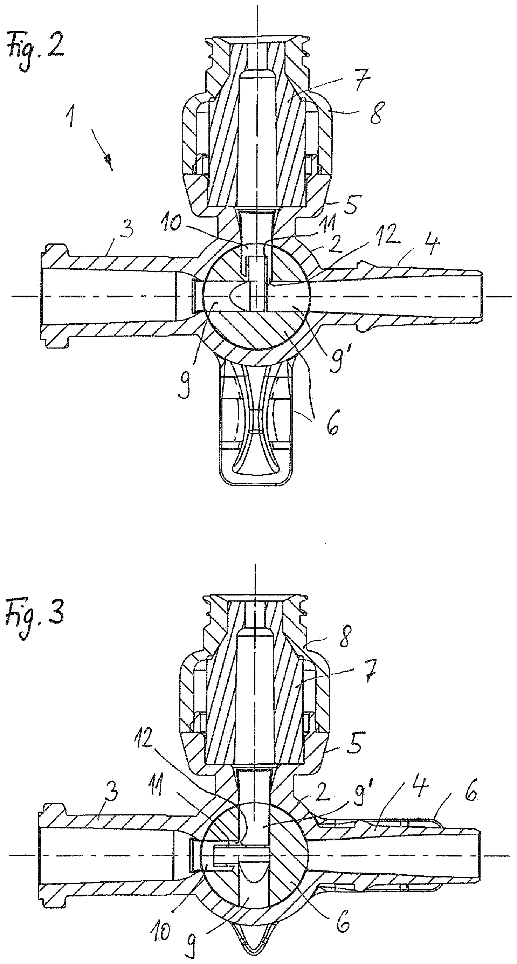

Um eine Durchspülung der Innenseite des Zuspritzports 5 im Bereich der Innenwandung des Zuspritzports 5 und einer Innenwandung der Verschlussmembran 7 zu verbessern, ist bei den Ausführungsformen gemäß den Fig. 1 bis 8 im Inneren des Stellorgans 6 im Bereich eines Strömungskanalabschnitts 10 ein mechanisches Strömungsbeschleunigungsmittel in Form eines in den Strömungskanalabschnitt 10 integrierten Rohrabschnitts 11 vorgesehen. Das Stellorgan 6 weist insgesamt drei Strömungskanalabschnitte 9, 9', 10 auf, die auf Höhe der Verbindungsporte 3, 4 und des Zuspritzports 5 in dem Stellorgan vorgesehen sind. Die Strömungskanalabschnitte 9, 10 dienen dazu, je nach Stellung des Stellorgans 6 zwischen den Verbindungsporten 3, 4 und dem Zuspritzport 5 Fluidverbindungen für eine entsprechende Fluiddurchströmung herzustellen. Unterschiedliche Stellpositionen des Stellorgans 6 relativ zum Fluidströmungsgehäuse 2 und damit unterschiedliche Fluidwege zwischen den Verbindungsporten 3, 4 und dem Zuspritzport 5 sind anhand der Fig. 2 bis 5 dargestellt.

In Fig. 2 ist eine Spülfunktion des Stellorgans 6 eingestellt. Dabei erstrecken sich die miteinander fluchtenden Strömungskanalabschnitte 9, 9' des Stellorgans 6 in koaxialer Verbindung geradlinig zwischen den gegenüberliegenden Verbindungsporten 3, 4, wohingegen der Strömungskanalabschnitt 10 rechtwinklig hierzu mit dem Inneren des Zuspritzports 5 koaxial fluchtet. Auch der Rohrabschnitt 11 ist in dieser Funktionsstellung zum Zuspritzport 5 hin geöffnet. Dabei ist erkennbar, dass der zum Verbindungsport 3 hin offene Strömungskanalabschnitt 9 des Stellorgans 6 rechtwinklig umgelenkt wird in den Rohrabschnitt 11, der einen kleineren Strömungsquerschnitt aufweist als der Strömungskanalabschnitt 9. Die Strömungskanalabschnitte 9 sowie der Strömungskanalabschnitt 10 des Stellorgans 6 weisen bei der dargestellten Ausführungsform zueinander identische Strömungsquerschnitte auf. Bei nicht dargestellten Ausführungsformen der Erfindung können die Strömungskanalabschnitte unterschiedliche Strömungsquerschnitte aufweisen, wobei insbesondere der Strömungsquerschnitt des Strömungskanalabschnittes 10 größer ist als der Strömungsquerschnitt des Strömungskanalabschnittes 9. Der Rohrabschnitt 11 ist einstückig in dem Stellorgan 6 ausgeformt, das vorzugsweise analog des Fluidströmungsgehäuses 2 aus einem Kunststoff, vorteilhaft in Form von Polycarbonat oder Polyamid, hergestellt ist. Der Rohrabschnitt 11 ist im Bereich seines Außenmantels zu einer Innenwandung des Strömungskanalabschnitts 10 beabstandet, wodurch sich zwischen dem Außenmantel des Rohrabschnitts 11 und der Innenwandung des Strömungskanalabschnitts 10 ein Ringspalt 12 ergibt. Über den Ringspalt 12 ist der Strömungskanalabschnitt 10 zu dem in Fig. 2 in Richtung des Verbindungsports 4 gewandten Strömungskanalabschnitt 9' hin offen. Der entsprechende Ringspalt 12 ermöglicht demzufolge eine Weiterleitung der durch den Rohrabschnitt 11 in das Innere des Zuspritzports 5 gespülten Fluiddurchströmung zum Verbindungsport 4. Der Strömungskanalabschnitt 9 ist hingegen zu dem gegenüberliegenden Strömungskanalabschnitt 9' hin verschlossen, so dass Fluid, das über den eingangsseitigen Verbindungsport 3 gemäß Fig. 2 zugeführt wird, ausschließlich über den Rohrabschnitt 11 in den Zuspritzport 5 geleitet und von dort über den Ringspalt 12 und den diametral gegenüberliegenden Strömungskanalabschnitt 9' zu dem ausgangsseitigen Verbindungsport 4 geführt werden kann.

In Fig. 3 ist das Stellorgan 6 relativ zum Fluidströmungsgehäuse 2 derart verdreht, dass der Rohrabschnitt 11 zum Verbindungsport 3 hin gerichtet ist, wohingegen der Strömungskanalabschnitt 9', der über den Ringspalt 12 zum Strömungskanalabschnitt 10 hin offen ist, zum Zuspritzport 5 hin gewandt ist. Der Verbindungsport 4 ist durch das Stellorgan 6 verschlossen.

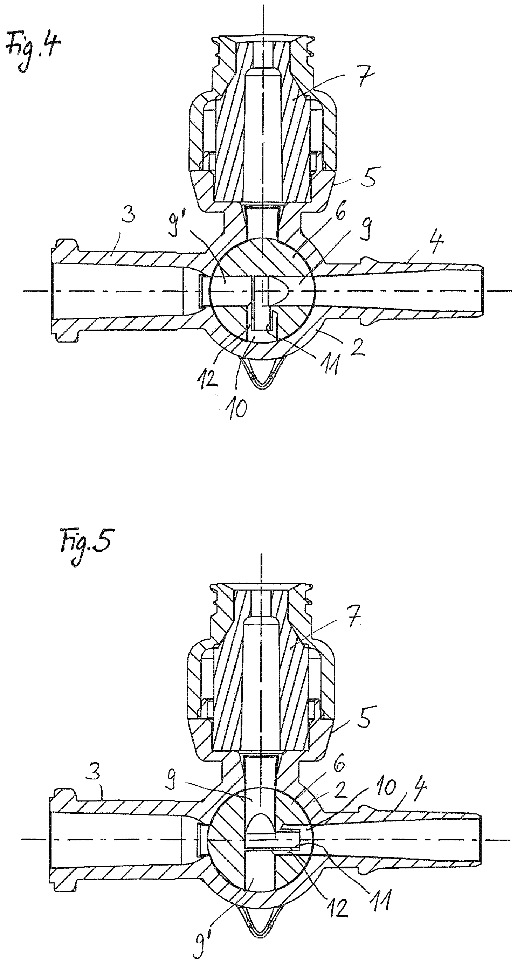

In Fig. 4 ist das Stellorgan 6 so verdreht, dass der Zuspritzport 5 geschlossen ist und sich ein koaxialer Fluiddurchgang zwischen den beiden Verbindungsports 3, 4 über die Strömungskanalabschnitte 9, 9' ergibt. Der Strömungskanalabschnitt 10 ist diametral gegenüberliegend zu dem Zuspritzport 5 im Fluidströmungsgehäuse 2 positioniert. Eine Fluiddurchströmung vom Verbindungsport 3 in Richtung des Verbindungsports 4 erfolgt daher über den Strömungskanalabschnitt 9', den Ringspalt 12 in den Strömungskanalabschnitt 10 und von dort über den Rohrabschnitt 11 in den anschließenden Strömungskanalabschnitt 9, der zum Verbindungsport 4 hin offen ist.

In Fig. 5 ist das Stellorgan 6 so gedreht, dass der Verbindungsport 3 geschlossen ist, wohingegen der eine Strömungskanalabschnitt 9 zum Zuspritzport 5 hin offen ist und der Strömungskanalabschnitt 10 zum Verbindungsport 4 geöffnet ist. Auch der Rohrabschnitt 11 ist koaxial zu einer Mittellängsachse des Verbindungsports 4 ausgerichtet. Diese Funktionsstellung des Stellorgans 6 entspricht einer Zuspritzstellung, in der über den Zuspritzport 5 eine Medikamentenlösung der Patientenleitung zugeführt werden kann.

Die Funktionsstellung nach Fig. 2 entspricht einer Spülstellung des Stellorgans 6, in der ein über den Verbindungsport 3 zugeführtes Fluid die Innenseite des Zuspritzports 5 und der Verschlussmembran 7 ausspülen kann und weitergeleitet wird zum ausgangsseitigen Verbindungsport 4, der mit der Patientenleitung verbunden ist.

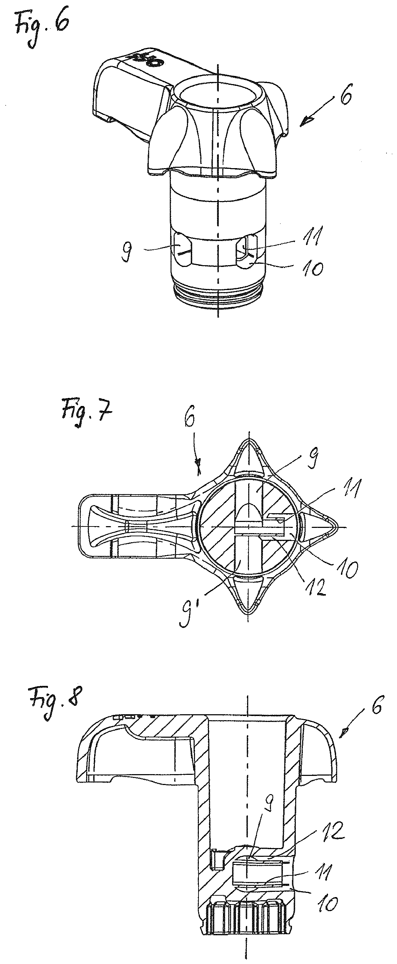

Anhand der Fig. 6 bis 8 ist das Stellorgan 6 ohne das Fluidströmungsgehäuse 2 und die entsprechenden Ports 3 bis 5 noch einmal dargestellt.

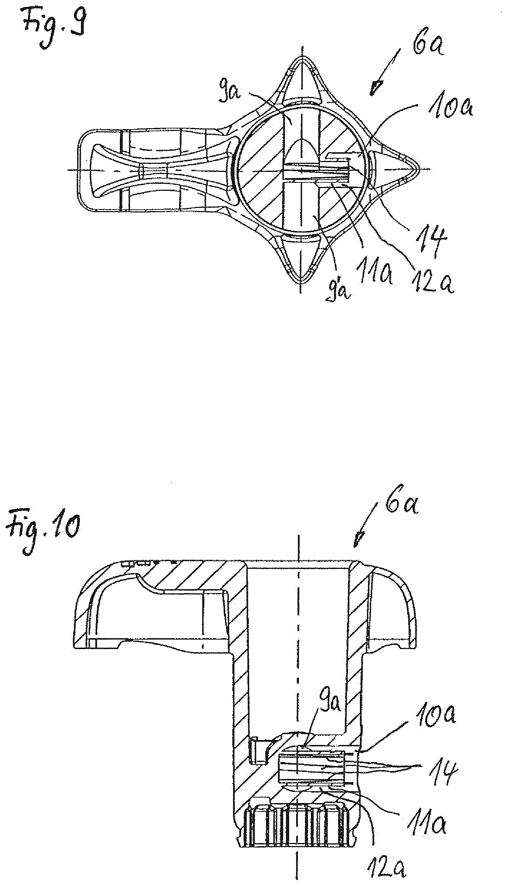

Das Stellorgan 6a nach den Fig. 9 und 10 entspricht im Wesentlichen dem zuvor bereits ausführlich beschriebenen Stellorgan 6 gemäß den Fig. 1 bis 8. Das Stellorgan 6a kann alternativ zu dem Stellorgan 6 in das Fluidströmungsgehäuse 2 des Dreiwegehahns 1 nach den Fig. 1 bis 8 eingesetzt werden. Wesentlicher Unterschied bei dem Stellorgan 6a ist es, dass der Rohrabschnitt 10a zusätzlich mit Drallerzeugungsgeometrien 14 im Bereich seiner Innenwandung versehen ist. Die Drallerzeugungsgeometrien 14 sind beim dargestellten Ausführungsbeispiel einstückig an entsprechenden Innenwandungsabschnitten des Rohrabschnitts 11a ausgeformt und bewirken eine zusätzliche Drallerzeugung auf die Fluiddurchströmung, die eine weitere Erhöhung der Strömungsgeschwindigkeit der Fluiddurchströmung zur Folge haben kann. Bei nicht dargestellten Ausführungsformen der Erfindung können Drallerzeugungsgeometrien an separat hergestellten Bauteilen vorgesehen sein, die in entsprechende Rohrabschnitte lösbar oder unlösbar eingebracht sind. Eine Durchspülung des Zuspritzports 5 kann hierdurch weiter verbessert werden. Die Drallerzeugungsgeometrien 14 sind insbesondere als wendelartig gestaltete Wandungsprofilierungen ausgeführt. Es können auch anders gestaltete Wandungsprofilierungen vorgesehen sein. Dadurch sollen äußere Strömungsbereiche der Fluiddurchströmung in Umfangsrichtung abgelenkt werden, wodurch insgesamt eine Drallfunktion für die Fluiddurchströmung erzielt wird.

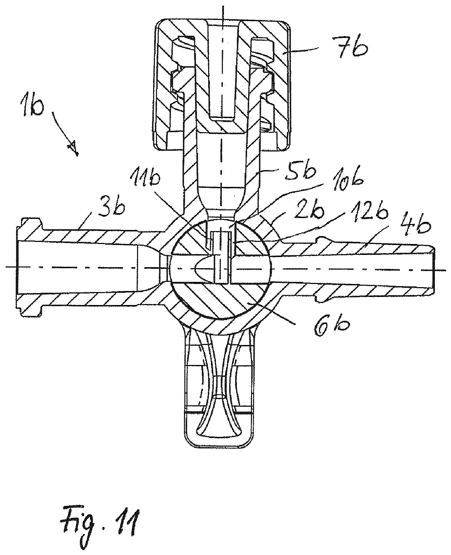

Die medizinische Fluidsteuervorrichtung gemäß Fig. 11 stellt einen Dreiwegehahn 1b dar, der im Wesentlichen identisch ausgeführt ist wie die anhand der Fig. 1 bis 8 beschriebene Ausführungsform. Zur Vermeidung von Wiederholungen wird daher auf die Offenbarung zu den Fig. 1 bis 8 verwiesen. Funktions- oder baugleiche Teile und Abschnitte des Dreiwegehahns 1b sind mit gleichen Bezugszeichen unter Hinzufügung des Buchstabens b versehen.

Einziger Unterschied des Dreiwegehahns 1b zu dem Dreiwegehahn 1 gemäß den Fig. 1 bis 8 ist es, dass der Zuspritzport 5b nicht mit einem Verschlusselement in Form einer Verschlussmembran, sondern vielmehr mit einem Verschlusselement in Form einer Verschlusskappe 7b versehen ist. Die Verschlusskappe 7b ist mit männlichen Luer-Lock-Anschlussprofilierungen versehen, wohingegen der Zuspritzport 5b mit komplementären weiblichen Luer-Lock-Anschlussprofilierungen ausgestaltet ist. Eine Bedienperson kann die Verschlusskappe 7b durch eine einfache Drehbewegung auf den Zuspritzport 5b aufschrauben oder von diesem entfernen, wobei ein Konusabschnitt der Verschlusskappe 7b dicht in eine Aufnahmeöffnung des Zuspritzports 5b eintaucht.

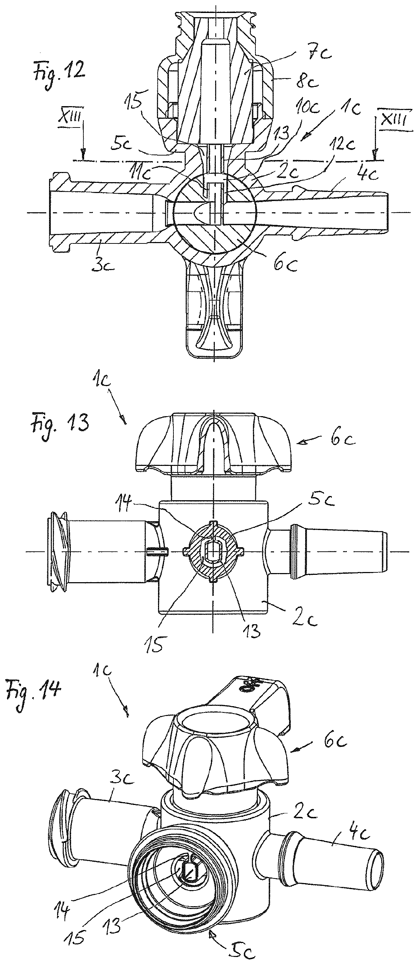

Bei der Ausführungsform nach den Fig. 12 bis 14 ist ein Dreiwegehahn 1c vorgesehen, der im Wesentlichen der zuvor anhand der Fig. 1 bis 8 beschriebenen Ausführungsform entspricht. Zur Vermeidung von Wiederholungen wird daher ergänzend auf die Offenbarung zu der Ausführungsform nach den Fig. 1 bis 8 verwiesen. Identische Teile und Abschnitte des Dreiwegehahns 1c sind mit gleichen Bezugszeichen unter Hinzufügung des Buchstabens c versehen. Nachfolgend wird auf die Unterschiede zu der Ausführungsform nach den Fig. 1 bis 8 eingegangen. Wesentlicher Unterschied bei der Ausführungsform nach den Fig. 12 bis 14 ist es, dass im Zuspritzport 5c ein ergänzendes mechanisches Strömungsbeschleunigungsmittel integriert ist, das als Rohrabschnitt 13 gestaltet ist. Der Rohrabschnitt 13 ist einstückig in einem Teilabschnitt des Fluidströmungsgehäuses 2c ausgeformt, der einen Teil des Zuspritzports 5c bildet. Dieser Teil des Zuspritzports 5c bildet einen Aufnahmestutzen für eine Befestigung des Aufnahmegehäuses 8c, wobei der Aufnahmestutzen und das Aufnahmegehäuse 8c die Verschlussmembran 7c umschließen. Der Rohrabschnitt 13 ist in einem Kanalabschnitt 15 des Aufnahmestutzens des Zuspritzports 5c integriert und einstückig mit einer Wandung des Kanalabschnittes 15 verbunden. Hierzu weist der Rohrabschnitt 13 an seiner Oberseite einen Verbindungssteg 14 auf, der den Rohrabschnitt 13 in dem Kanalabschnitt 15 des Zuspritzports 5c aufhängt. Der Rohrabschnitt 13 wird gemeinsam mit dem Kanalabschnitt 15 und dem Verbindungssteg 14 sowie dem Fluidströmungsgehäuse 2c in einem entsprechenden Herstellungsverfahren, insbesondere durch ein Kunststoffspritzgussverfahren, ausgeformt und ist über den Verbindungssteg 14 einstückig mit dem Fluidströmungsgehäuse 2c verbunden. An dem Fluidströmungsgehäuse 2c ebenfalls einstückig angeformt sind die Verbindungsports 3c, 4c. Ein Rohrquerschnitt des Rohrabschnittes 13 ist identisch zu dem Rohrquerschnitt des Rohrabschnittes 11c, der in dem Stellorgan 6c ausgeformt ist. Die beiden Rohrabschnitte 11c und 13 weisen identische Strömungsquerschnitte zueinander auf. Anhand der Fig. 13 ist erkennbar, dass der Rohrabschnitt 13 einen rotationsunsymmetrischen Querschnitt aufweist. Der Rohrabschnitt 13 wie auch der Rohrabschnitt 11c, der den Rohrabschnitten 11 und 11b gemäß den Fig. 1 bis 11 entspricht, weisen zwei einander horizontal gegenüberliegende, zueinander parallele Wandungsabschnitte auf, die oben und unten jeweils in zwei in Querschnitt bogenförmig gewölbte Wandungsabschnitte übergehen. Auch einen rotationssymetrischen Querschnitt (Kreis) berücksichtigen. Die Höhenerstreckung des Strömungsquerschnittes ist - wie anhand der Fig. 13 erkennbar ist - größer als eine Quererstreckung des Strömungsquerschnittes des jeweiligen Rohrabschnittes 13 oder 11, 11a, 11b, 11c. Der Rohrabschnitt 13 kann gemäß nicht dargestellten Ausführungsformen der Erfindung auch über mehrere über seinen Umfang verteilt angeordnete Verbindungsstege mit dem Aufnahmestutzen des Zuspritzports 5c einstückig verbunden sein. Alternativ wird der Rohrabschnitt 13 zwar über einen einzelnen Verbindungssteg im Kanalabschnitt 15 des Zuspritzports 5c gehalten, der bei einer entsprechend anderen Ausführungsform der Erfindung aber nicht im Bereich der Oberseite, sondern im Bereich einer Unterseite oder im Bereich einer gemäß Fig. 13 nach links oder rechts weisenden Außenseite positioniert ist.

## Patentansprüche

1. Medizinische Fluidsteuervorrichtung für ein medizinisches Fluidleitungssystem mit einem Fluidströmungsgehäuse (2, 2b, 2c), das mit wenigstens einem Verbindungsport (3, 3b, 3c; 4, 4b, 4c) zur Verbindung mit einer weiteren Funktionskomponente des Fluidleitungssystems versehen ist, und das einen Zuspritzport (5, 5b, 5c) aufweist, der mit einem öffnungsfähigen Verschlusselement (7, 7b, 7c) versehen ist, sowie mit einem Stellorgan (6, 6a, 6b, 6c), das in dem Fluidströmungsgehäuse (2, 2b, 2c) beweglich gelagert ist und Strömungskanalabschnitte (9, 9', 10; 9a, 9'a, 10a; 10b; 10c) aufweist, die abhängig von einer Stellposition des Stellorgans (6, 6a, 6b, 6c) dem wenigstens einen Verbindungsport (3, 3b, 3c; 4, 4b, 4c) und/oder dem Zuspritzport (5, 5b, 5c) für eine Fluiddurchströmung zustellbar sind, wobei wenigstens einem Strömungskanalabschnitt (10, 10a, 10b, 10c) des Stellorgans (6, 6a, 6b, 6c) und/oder dem Zuspritzport (5c) wenigstens ein mechanisches Strömungsbeschleunigungsmittel (11, 11a, 11b, 11c; 13) zugeordnet ist, das bei einer Fluiddurchströmung eine innenseitige Spülung des Zuspritzports (5, 5b, 5c) unterstützt, **dadurch gekennzeichnet, dass** das mechanische Strömungsbeschleunigungsmittel durch einen in den Strömungskanalabschnitt (10, 10a, 10b, 10c) und/oder den Zuspritzport (5c) integrierten Rohrabschnitt (11, 11a, 11b, 11c; 13) gebildet ist, dessen Strömungsquerschnitt kleiner ist als ein Strömungsquerschnitt eines stromaufwärts benachbarten Strömungskanalabschnitts (10, 10a, 10b, 10c) des Stellorgans (6, 6a, 6b, 6c) oder des Zuspritzports (5c)oder des Verbindungsports (3, 4; 3b, 4b; 3c, 4c), und dass zwischen einer Außenwandung des Rohrabschnitts (11, 11a, 11b, 11c; 13) und einer Wandung des umgebenden Strömungskanalabschnitts (10, 10a, 10b, 10c; 15) oder einer Innenwandung des Zuspritzports (5c) ein eine Fluiddurchströmung ermöglichender Ringspalt (12, 12a) vorgesehen ist.

2. Medizinische Fluidsteuervorrichtung nach Anspruch1, **dadurch gekennzeichnet, dass** der Rohrabschnitt (11, 11a, 11b, 11c; 13) einstückig in dem Strömungskanalabschnitt (10, 10a, 10b, 10c) des Stellorgans (6, 6a, 6b, 6c) oder dem Zuspritzport (5c) des Fluidströmungsgehäuses (2, 2b, 2c) ausgeformt ist.

3. Medizinische Fluidsteuervorrichtung nach Anspruch 1 oder 2, **dadurch gekennzeichnet, dass** eine Innenwandung des Rohrabschnitts (11a) mit Drallerzeugungsgeometrien (14) versehen ist, die auf die Fluiddurchströmung einwirken.

## Claims

1. Medical fluid control device for a medical fluid line system, comprising a fluid flow housing (2, 2b, 2c) which is provided with at least one connection port (3, 3b, 3c; 4, 4b, 4c) for connecting to a further functional component of the fluid line system and which has an injection port (5, 5b, 5c) which is provided with an openable closure element (7, 7b, 7c), and also comprising an adjustment member (6, 6a, 6b, 6c) which is mounted movably in the fluid flow housing (2, 2b, 2c) and which has flow channel sections (9, 9', 10; 9a, 9'a, 10a; 10b; 10c) which, in dependence on an adjustment position of the adjustment member (6, 6a, 6b, 6c), can be moved toward the at least one connection port (3, 3b, 3c; 4, 4b, 4c) and/or the injection port (5, 5b, 5c) for a fluid throughflow, wherein assigned to at least one flow channel section (10, 10a, 10b, 10c) of the adjustment member (6, 6a, 6b, 6c) and/or to the injection port (5c) is at least one mechanical flow acceleration means (11, 11a, 11b, 11c; 13) which assists with inside flushing of the injection port (5, 5b, 5c) during a fluid throughflow, **characterized in that**
the mechanical flow acceleration means is formed by a tube section (11, 11a, 11b, 11c; 13) which is integrated into the flow channel section (10, 10a, 10b, 10c) and/or into the injection port (5c) and whose flow cross section is smaller than a flow cross section of a flow channel section (10, 10a, 10b, 10c), adjacent upstream, of the adjustment member (6, 6a, 6b, 6c) or of the injection port (5c) or of the connection port (3, 4; 3b, 4b; 3c, 4c), and **in that** an annular gap (12, 12a) allowing a fluid throughflow is provided between an outer wall of the tube section (11, 11a, 11b, 11c; 13) and a wall of the surrounding flow channel section (10, 10a, 10b, 10c; 15) or an inner wall of the injection port (5c).

2. Medical fluid control device according to claim 1, **characterized in that** the tube section (11, 11a, 11b, 11c; 13) is formed integrally in the flow channel section (10, 10a, 10b, 10c) of the adjustment member (6, 6a, 6b, 6c) or in the injection port (5c) of the fluid flow housing (2, 2b, 2c).

3. Medical fluid control device according to claim 1 or 2, **characterized in that** an inner wall of the tube section (11a) is provided with swirl-generating geometries (14) which act on the fluid throughflow.

## Revendications

1. Dispositif médical de commande de fluide pour un système médical de conduite de fluide, comprenant un logement d'écoulement de fluide (2, 2b, 2c) qui est doté d'au moins un port de liaison (3, 3b, 3c ; 4, 4b, 4c) pour la liaison à un autre composant fonctionnel du système de conduite de fluide et qui présente un port d'injection (5, 5b, 5c) qui est doté d'un élément d'obturation (7, 7b, 7c) pouvant s'ouvrir, ainsi que comprenant un organe de réglage (6, 6a, 6b, 6c) qui est monté mobile dans le logement d'écoulement de fluide (2, 2b, 2c) et qui présente des sections de canal d'écoulement (9, 9', 10 ; 9a, 9'a, 10a ; 10b ; 10c) qui peuvent être approchées, en fonction d'une position de réglage de l'organe de réglage (6, 6a, 6b, 6c), dudit au moins un port de liaison (3, 3b, 3c ; 4, 4b, 4c) et/ou du port d'injection (5, 5b, 5c) pour un passage de fluide, dans lequel au moins un moyen mécanique d'accélération d'écoulement (11, 11a, 11b, 11c ; 13) est associé à au moins une section de canal d'écoulement (10, 10a, 10b, 10c) de l'organe de réglage (6, 6a, 6b, 6c) et/ou au port d'injection (5c), ledit moyen assistant un rinçage côté intérieur du port d'injection (5, 5b, 5c) lors d'un passage de fluide, **caractérisé en ce que** le moyen mécanique d'accélération d'écoulement est formé par une section de tuyau (11, 11a, 11b, 11c ; 13) intégrée dans la section de canal d'écoulement (10, 10a, 10b, 10c) et/ou le port d'injection (5c), dont la section transversale d'écoulement est inférieure à une section transversale d'écoulement d'une section de canal d'écoulement voisine en amont (10, 10a, 10b, 10c) de l'organe de réglage (6, 6a, 6b, 6c) ou du port d'injection (5c) ou du port de liaison (3, 4 ; 3b, 4b ; 3c, 4c), et **en ce qu'**une fente annulaire (12, 12a) permettant un passage de fluide est prévue entre une paroi extérieure de la section de tuyau (11, 11a, 11b, 11c ; 13) et une paroi de la section de canal d'écoulement environnante (10, 10a, 10b, 10c ; 15) ou une paroi intérieure du port d'injection (5c).

2. Dispositif médical de commande de fluide selon la revendication 1, **caractérisé en ce que** la section de tuyau (11, 11a, 11b, 11c ; 13) est formée d'une seule pièce dans la section de canal d'écoulement (10, 10a, 10b, 10c) de l'organe de réglage (6, 6a, 6b, 6c) ou le port d'injection (5c) du logement d'écoulement de fluide (2, 2b, 2c).

3. Dispositif médical de commande de fluide selon la revendication 1 ou 2, **caractérisé en ce qu'**une paroi intérieure de la section de tuyau (11a) est munie de géométries de génération de torsion (14) qui agissent sur le passage de fluide.
